(19) Europäisches Patentamt
European Patent Office
Office européen des brevets

(11) Publication number: **0 050 440 A1**

(12) # EUROPEAN PATENT APPLICATION

(21) Application number: **81304619.0**

(22) Date of filing: **06.10.81**

(51) Int. Cl.³: **C 07 C 149/46**, A 01 N 31/14, A 01 N 31/02

(30) Priority: **18.10.80 GB 8033696**

(43) Date of publication of application: **28.04.82 Bulletin 82/17**

(84) Designated Contracting States: **AT BE CH DE FR GB IT LI LU NL SE**

(71) Applicant: **FBC LIMITED, Hauxton, Cambridge CB2 5HU (GB)**

(72) Inventor: **Garrod, John Frederick, 2 Maltings Cottages The End Way, Steeple Bumpstead Haverhill Suffolk (GB)**
Inventor: **Greenwood, Douglas, 14 The Paddocks, Cambridge CB1 3HG (GB)**
Inventor: **Van den Oudenweyer, Jacobus Gerardus C. J. M. W., 29 Westfield Little Abington, Cambridgeshire CB1 6BE (GB)**

(74) Representative: **Waldman, Ralph David et al, Industrial Property Department FBC Limited Chesterford Park Research Station, Saffron Walden, Essex CB10 1XL (GB)**

(54) **Sulphonium compounds and herbicidal compositions containing them.**

(57) A herbicidal composition comprising a compound of formula I

$$Ph\text{-}O\text{-}CH_2CH_2S(CH_3)_2X^- \quad I$$

where Ph is phenyl and $X^-$ is an equivalent of an anion in admixture with an agronomically acceptable diluent or carrier. The compounds are particularly active against yellow and purple nutsedges.

EP 0 050 440 A1

SULPHONIUM COMPOUNDS AND HERBICIDAL COMPOSITIONS CONTAINING THEM.



This invention relates to products having herbicidal properties.

According to the invention there is provided a herbicidal composition comprising a compound of formula I

$$Ph\text{-}O\text{-}CH_2CH_2\overset{+}{S}(CH_3)_2X^- \qquad I$$

where Ph is phenyl and $X^-$ is an equivalent of an anion, in admixture with an agronomically acceptable diluent or carrier.

Many of the compounds of formula I are novel and the invention therefore includes compounds of formula I where $X^-$ has the meaning given above, except iodide.

X is preferably a monovalent group and is preferably selected from halogen, e.g. chlorine, iodine or bromine, optionally substituted alkylsulphate, e.g. methylsulphate, optionally substituted alkylsulphonate e.g. methanesulphonate and trifluoromethanesulphonate; arylsulphonate, e.g. benzenesulphonate and p-toluenesulphonate; and tetrafluoroborate.

The compounds of the invention have herbicidal activity and in particular are active against yellow and purple nutsedge, (Cyperus esculentus and Cyperus rotundus respectively). The latter weed has been classified as the world's worst weed in the book "The World's Worst Weeds"

published in 1977 by the University Press of Hawaii.

The compounds of the invention are generally active as post-emergent herbicides and have little or no adverse effect at normal herbicidal rates on many crops in which nutsedges are a problem, e.g. maize, cotton, sugar cane, rice, soybeans, peanuts and turf. Because of the highly selective post-emergent activity, the compounds need only be applied when the weeds can be seen to be a problem and hence unnecessary application is avoided, which can be the case with a pre-emergent herbicide.

The diluent or carrier for the compositions of the invention can be a solid or a liquid, and may comprise a surface active agent. The active compound can be employed in a wide variety of formulations, for example, a solution, a dispersion, a soluble powder, a dispersible powder or a granule.

Preferred formulations are solutions and soluble powders. Solutions, preferably in water, can be of an appropriate concentration for direct application to the crop or they can be in the form of a concentrated primary composition, for example, a concentrated aqueous solution which requires dilution with a suitable quantity of water or other diluent before application. A concentrated aqueous solution can also contain a small quantity of a suitable agent to prevent foaming.

Such concentrated primary compositions are a convenient way of supplying the consumer, and a further example is a soluble powder formulation which comprises the active compound in powder form optionally together with a humectant or flow agent, for example silica.

As a dispersion, the composition of the invention comprises an active compound dispersed in a liquid medium together with a dispersing agent. The dispersant may be a high boiling hydrocarbon usually in association with a suitable wetting agent.

A granular solid comprises an active compound associated with a solid powdery diluent, for example kaolin, the mixture being granulated by known methods.

The concentration of the active compound in the composition of the invention can vary widely. In the case of a concentrated primary composition it is preferably from 15 to 99 per cent by weight. For example a concentrated aqueous solution may contain from 10 to 50 per cent by weight the concentration depending on the solubility of the compound, and a soluble powder will preferably contain 50 to 95 per cent by weight. The concentration of active compound in a composition intended for direct application preferably comprises from 0.001 to 10 per cent, more especially from 0.005 to 6 per cent by weight, although when aerial spraying of a crop is

contemplated compositions having a higher concentration, for instance, up to 30 per cent by weight may be chosen in preference.

The compounds of the invention may be used in admixture or in combination with other herbicides and especially post-emergent herbicides used to control broad-leaved and/or grassy weeds. Examples of suitable herbicides include phenoxyalkanoic acids, e.g. 2,4-D, 2,4-DB, dichlorprop, MCPA, mecoprop and MCPB; phenoxyphenoxyalkanoic acids, e.g. diclofop-methyl and difenopenten; pyridyloxyphenoxyalkanic acids, e.g. fluometuron and chloroxuron; triazines, e.g. atrazine cyanazine and prometryne; benzoic acids, e.g. dicamba; ethers, e.g. bifenox and acifluorofen; phophorus compounds, e.g. glyphosate and compounds of the type described in EPO specificatin 30424 (and especially salts of 4-(hydroxymethylphosphinyl)-2-oxobutanoic acid); bipyridyl compounds e.g. paraquat; dinitrophenols, e.g. dinoseb and its acetate; picodinic acids, e.g. 3,6-dichloropicolinic acid; methylarsonic acid (MSMA); mefluidide; bentazone; methazole; norflurazon; alloxydim sodium; sethoxydim; and DPX 4189.

We have also found that the activity of compounds of the invention can often be enhanced by the addition of surface-active agents in amounts which are usually higher

than are normally present in commercial pesticidal compositions. The surface active agent is generally nonionic although a blend of surface-active agents may be used in which case at least one of the blend is preferably nonionic. The weight ratio of surface-active agent to the compound of formula I is preferably in the range 10 : 1 to 1 : 10 e.g. 1 : 1 to 1 : 5.

The novel compounds of the invention can be prepared by a) reacting methyl 2-phenoxyethyl sulphide with an alkylating agent of formula, MeY, where Y is one equivalent of the anion of a strong acid (except iodide), e.g. sulphate or p-toluenesulphonate, or b) treating dimethyl-2-phenoxyethylsulphonium iodide to a double decomposition reaction to replace the iodide by another anion. If it is desired, salts produced by the above processes can be converted to other salts (except to the iodide) by a double decomposition reaction. The double decomposition reaction may be a precipitation reaction in which for example, a concentrated aqueous solution of the sulphonium methyl sulphate is reacted with a concentrated aqueous solution of an alkali metal salt or for example, in the case of the preparation of the chloride, with an aqueous suspension of silver chloride. The first reaction can be carried out in the presence of an organic solvent, for example nitrobenzene, although this can be dispensed

with when the alkylating agent is itself a solvent. Preferably the reactions are performed at a temperature of from 10° to 120°C.

The invention is illustrated in the following Examples. Structures of novel compounds were confirmed by elementary and other appropriate analyses.

Examples 1-3

Methyl 2-phenoxyethyl sulphide (5.04 g) was mixed with methyl *p*-toluenesulphonate (6.0 g) and allowed to stand for 6 days. The mixture was triturated with ether and the product recrystallised from ethanol to give dimethyl-2-phenoxyethylsulphonium p-toluenesulphonate, m.p. 145-147°C.

In a similar manner dimethyl sulphate gave dimethyl-2-phenoxyethylsulphonium methyl sulphate, m.p. 94-96°C, (Example 2) and methyl trifluoromethane sulphonate in ether gave dimethyl-2-phenoxyethylsulphonium trifluoromethanesulphonate, m.p. 103°C (Example 3).

Example 4

A solution of the product of Example 2 (7.4 g) in water (7.4 ml.) was mixed with a solution of sodium tetrafluoroborate (7.4 g.) in water. The product was filtered, washed with ice-water and recrystallised from isopropanol to give dimethyl-2-phenoxyethylsulphonium tetrafluoroborate, m.p. 72-76°C.

Example 5

A solution of dimethyl-2-phenoxyethylsulphonium iodide (J.C.S. 1947, 766) (20 g.) in water (400 ml.) was mixed with silver chloride (freshly prepared from silver nitrate (34 g.) and sodium chloride (11.7 g.)). The mixture was stirred for 23 hours and filtered whilst washing with water. The filtrate was evaporated to dryness at 20-30°C and the residue dried *in vacuo* to give dimethyl-2-phenoxyethylsulphonium chloride, obtained as crystalline solid which was too deliquescent for a melting point determination.

Example 6

Tubers of purple nutsedge (*Cyperus rotundus*) were planted in John Innes potting compost in 9 cm plastic pots and allowed to grow in in the glasshouse. When the young plants had 3-4 leaves and were 5-7 cm tall they were selected for uniformity and sprayed with aqueous solutions of the compound under study at various rates. There were three replicates per treatment.

Damage to the plant was assessed after three weeks using a scale of 0 to 4 where 0 is no effect and 4 is complete kill. At a rate equivalent to 8 kg/hectare the product of Example 5 gave a score of 2, the products of Examples 2 and 4 gave a score of 3 and the products of Examples 1 and 3 gave a score of 4. Dimethyl-2-phenoxyethyl sulphonium iodide (the starting material of Example 5) also gave a score of 3.

Example 7

A powder formulation was made as follows.

| | % w/w |
|---|---|
| Compound of Example 1 | 95 |
| Sodium dioctylsulphosuccinate | 0.1 |
| Silica to | 100 |

Example 8

The formulation of Example 7 was diluted with water and applied to purple nutsedge (Cyperus rotundus) and yellow nutsedge (Cyperus esculentus) grown as in Example 6. Product was applied at the 2-3 leaf stage. Four replicates were used for each treatment. Three weeks after applying to yellow nutsedge a rate of 8 kg/hectare gave 100% control. The same rate gave 98% control of the purple nutsedge after four weeks.

Example 9

Example 8 was repeated in a similar manner. Tests were carried out in which 0.5% by volume of further surfactants were added to the formulaton.

The results are shown below.

Yellow nutsedge : 6 weeks after spraying

| | % Control | | |
|---|---|---|---|
| kg of compound of Ex 1/ hectare | without extra surfactant | + Tween 20[1] | + Agral 90[2] |
| 1.0 | 4 | 83 | 70 |
| 2.0 | 50 | 93 | 95 |
| 4.0 | 91 | 97 | 99 |

[1] A Sorbitan ester ethoxylate

[2] A nonylphenolethoxylate

<u>Purple nutsedge</u> : 4 weeks after spraying

| | % Control | | |
|---|---|---|---|
| kg of compound of Ex 1/ hectare | without extra surfactant | + Tween 20[1] | + Agral 90[2] |
| 1.0 | 4 | 75 | 90 |
| 2.0 | 53 | 91 | 94 |
| 4.0 | 88 | 98 | 99 |

It will be seen that the extra surfactant (which is present in relatively much higher amounts than the small amount of sodium dioctylsulphosuccinate) results in a significant enhancement of the activity of the compound of Example 1.

CLAIMS

1. A herbicidal composition comprising a compound of formula I

$$Ph\text{-}O\text{-}CH_2CH_2\overset{+}{S}(CH_3)_2X^- \qquad I$$

where Ph is phenyl and $X^-$ is an equivalent of an anion, in admixture with an agronomically acceptable diluent or carrier.

2. Compounds of formula I

$$Ph\text{-}O\text{-}CH_2CH_2\overset{+}{S}(CH_3)_2X^- \qquad I$$

where Ph is phenyl and $X^-$ is an equivalent of an anion, except iodide.

3. Dimethyl-2-phenoxyethylsulphonium p-toluenesulphonate.

4. A process for preparing a compound of formula I

$$Ph\text{-}O\text{-}CH_2CH_2\overset{+}{S}(CH_3)_2X^- \qquad I$$

where Ph is phenyl and $X^-$ is an equivalent of an anion other than iodide which comprises a) reacting methyl 2-phenoxyethyl sulphide with an alkylating agent of formula MeY, where Y is one equivalent of the anion of a strong acid, or b) treating

dimethyl-2-phenoxyethylsulphonium iodide to a double decomposition reaction to replace the iodide by another anion, and c) where desired treating a salt produced under (a) or (b) to a double decomposition reaction to convert it to another salt (except the iodide)

5. A process according to claim 4 in which $X^-$ is p-toluenesulphonate.

6. A process according to claim 4 or 5 in which the reactions are carried out at a temperature of 10 to 120°C.

European Patent Office

0050440

# EUROPEAN SEARCH REPORT

Application number
EP 81 30 4619

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| X | JOURNAL OF THE AMERICAN CHEMICAL SOCIETY, vol.55, December 1933 R.R. RENSHAW et al.:"Basis for the Physiological Activity of Onium Compounds. XV. Sulfonium Compounds 1,2" pages 4951-4953 * table I, 4th compound * | 2-6 | C 07 C 149/46<br>A 01 N 31/14<br>31/02 |
| D | JOURNAL OF THE CHEMICAL SOCIETY, 1947 LONDON (GB) C.W. CRANE et al.:"The Alkaline Fission of Some 2-Substituted Dimethylethylsulphonium Iodides" pages 766-772 * page 767 * | 2,3 | |

TECHNICAL FIELDS SEARCHED (Int.Cl. 3)

C 07 C 149/46
A 01 N 31/14

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 12-01-1982 | GRAMAGLIA |

EPO Form 1503.1 06.78